# EUROPÄISCHE PATENTSCHRIFT

(11) **EP 1 397 083 B1**
(45) Veröffentlichungstag und Bekanntmachung des Hinweises auf die Patenterteilung: **06.09.2006**
(21) Anmeldenummer: 02754727.2
(22) Anmeldetag: 20.06.2002
(51) Int. Cl.: A61B 18/00

(54) **VERFAHREN UND VORRICHTUNG FÜR DIE PLASMACHIRURGIE**
METHOD AND DEVICE FOR PLASMA SURGERY
PROCEDE ET DISPOSITIF DE CHIRURGIE PLASMA

(30) Priorität: 22.06.2001 DE 10129685
(43) Veröffentlichungstag der Anmeldung: 17.03.2004
(73) Patentinhaber: Erbe Elektromedizin GmbH, 72072 Tübingen (DE)
(72) Erfinder: FARIN, Günter, 72072 Tübingen (DE)
(74) Vertreter: Bohnenberger, Johannes
(86) Internationale Anmeldenummer: PCT/EP2002/006858
(87) Internationale Veröffentlichungsnummer: WO 2003/000150

(56) Entgegenhaltungen:
- DE-B- 1 159 574
- US-A- 2 828 748
- US-A- 5 330 469
- US-B1- 6 197 026
- US-B1- 6 206 878

## Beschreibung

Die Erfindung betrifft ein Verfahren und Vorrichtung für die Plasmachirurgie.

Die Plasmachirurgie ist ein Verfahren der Hochfrequenzchirurgie, bei welchem hochfrequenter elektrischer Wechselstrom (HF-Strom) durch elektrisch ionisiertes und folglich elektrisch leitfähiges Edelgas (Plasma), insbesondere Argon (Argon-Plasma) oder Helium (Helium-Plasma), in das zu behandelnde Zielgewebe appliziert wird um in diesem Zielgewebe chirurgisch relevante, thermische Effekte zu erzeugen. Das bisher am meisten hierfür verwendete Edelgas ist Argon und der bekannteste thermische Effekt im Gewebe ist die Coagulation, weswegen dieses Verfahren auch unter der Bezeichnung "Argon-Plasma-Koagulation (APC)" bekannt ist. Prinzipiell können statt einem Edelgas auch andere Gase oder Gasgemische für die Plasmachirurgie angewendet werden. So wird beispielweise seit über 50 Jahren die Luft als Gas genutzt, was unter der Bezeichnung Fulguration oder Spray-Koagulation bekannt ist. Edelgase haben jedoch den Vorteil, daß sie keine chemischen Effekte verursachen bzw. sich chemisch neutral verhalten. Dieser Vorteil kann insbesondere bei endoskopischer Anwendung der Plasmachirurgie genutzt werden, wo die bei Anwendung von Luft unvermeidliche Rauchbildung die Sicht behindern würde und die bei Anwendung von Luft unvermeidliche Verbrennung bzw. Vaporisation biologischer Gewebe schwere Komplikationen, wie beispielsweise Perforationen von Organwänden verursachen könnte.

In der offenen sowie in der minimal invasiven Chirurgie wird die Plasmachirurgie seit über 20 Jahren vorwiegend zur thermischen Blutstillung angewendet, wobei diese Blutstillung auf die thermische Koagulation des biologischen Gewebes zurückgeführt wird. Hierbei wird entweder die bereits vorhandene Luft angewendet - was dann mit "Fulguration" oder "Spray Koagulation" bezeichnet wird, oder es wird ein Edelgas, vorwiegend Argon, angewendet, was dann mit "Argon-Plasma-Coagulation (APC)" bezeichnet wird.

Seit der Entwicklung katheterförmiger, flexibler Instrumente für die APC, welche durch einen Arbeits- bzw. Instrumentierkanal flexibler Endoskope anwendbar sind (G. Farin, K.E. Grund: Technologie of Argon Plasma Coagulation with Particular Regard to Endoscopic Applications. Endoscopic Surgery and Allied Technologies, No. 1, Vol. 2, Feb. 1994: 71-77), kann die APC auch in der flexiblen Endoskopie angewendet werden. Hier hat die APC rasch ein breites Indikationsspektum gefunden. Außer zur thermischen Blutstillung wird die APC in der flexiblen Endoskopie u.a. auch zur thermischen Destruktion bzw. thermischen Devitalisierung pathologischer Gewebe angewendet (Grund K.E., Storek D, Farin G: Endoscopic Argon Plasma Coagulation (APC) - First Clinical Experiences in Flexible Endoskopie. Endoscopic Surgery and Allied Technologies, No. 1, Vol. 2, Feb. 1994: 42-46). Obwohl die Bezeichnung Argon-Plasma-Coagulation (APC) für dieses Verfahren in der flexiblen Endoskopie unter Berücksichtigung neuerer Indikationen zu eng ist und der Terminus "Plasmachirurgie" geeigneter wäre, wird im folgenden weiterhin die Bezeichnung Argon-Plasma-Coagulation (APC) für dieses Verfahren verwendet, ohne dieses Verfahren jedoch auf das Edelgas "Argon" sowie auf den thermischen Effekt "Koagulation" zu beschränken.

Ein bekanntes Problem der Anwendung thermischer Verfahren im Gastrointestinaltrakt sowie im Tracheobronchialsystem und im Bereich des Rachens ist das unbeabsichtigte Zünden brennbarer oder gar explosibler Stoffe. Im Gastrointestinaltrakt können brennbare endogene Gase vorhanden sein, welche durch die relativ hohe Temperatur des Plasmas zündbar sind. Im Tracheobronchialsystem können brennbare Stoffe bei hoher Sauerstoffkonzentration des Atemgases oder gar bei Beatmung des Patienten mit reinem Sauerstoff durch die relativ hohe Temperatur des Plasmas gezündet werden und in der hohen Sauerstoffkonzentration intensives Feuer verursachen. Es sind mehrere entsprechende Schadenfälle ohne und mit letalem Ausgang bekannt. Obwohl Edelgase kein Feuer verursachen können sind Schadenfälle durch Feuer und/oder Explosionen auch bei Anwendung der APC bekannt.

Aus der DE 41 39 029 A1 ist eine Vorrichtung nach dem Oberbegriff des Patentanspruches 1 bekannt. Bei dieser Vorrichtung treten die eingangs erläuterten Probleme auf.

Aus der DE 11 59 574 B ist eine Sicherheitsvorrichtung für Hochfrequenz-Energieapparate bekannt, bei der eine Steuerung vorgesehen ist, welche ein Inert-Gas so lange durch ein Lumen eines angeschlossenen Instrumentes strömen lässt, bis sichergestellt ist, dass das Lumen frei von brennbaren Gasen ist.

Der Erfindung liegt die Aufgabe zu Grunde, eine Vorrichtung bzw. ein Verfahren der eingangs genannten Art dahin gehend weiter zu bilden, dass ein Zünden brennbarer Stoffe vermieden wird.

Diese Aufgabe wird durch eine Einrichtung nach Anspruch 1 gelöst.

Weiterhin wird die Aufgabe durch ein Verfahren zum Betreiben einer Einrichtung für die Argon-Plasma-Koagulation gemäß Anspruch 8 gelöst.

Um die zeitliche Verzögerung Δt der Aktivierung der APC durch den Argon-Vorlauf möglichst kurz zu halten, ist es zweckmäßig, hierfür eine möglichst hohe Argon Flow-Rate zu realisieren, welche in der Regel höher (hoch) sein kann als die für die APC erforderliche Argon Flow-Rate (niedrig). Hierbei kann es vorteilhaft sein, die Menge des Argon-Vorlaufs von relevanten Randbedingungen abhängig zu machen, welche entweder vor der Anwendung der APC manuell eingestellt werden oder durch automatische Erfassung dieser Randbedingungen automatisch gesteuert oder geregelt werden. So kann beispielsweise der Argon-Vorlauf proportional zur Sauerstoffkonzentration des Beatmungsgases eingestellt oder automatisch gesteuert bzw. geregelt werden. So kann der Argon-Vorlauf außerdem proportional zur Dauer der Pause zwischen zwei oder mehreren Aktivierungen und/oder der Dauer der Aktivierungen der APC eingestellt oder gesteuert bzw. geregelt werden.

Eine zur Lösung dieser Aufgabe geeignete Vorrichtung besteht aus einer Argon-Quelle Ar, z.B. Argon Gasflasche, welche mit mindestens einem steuerbaren Gas-Ventil V sowie mit mindestens einem Gas-Flow-Sensor SE ausgestattet ist, an welchen der Argon Vorlauf sowie die für die APC erforderliche Argon Flow-Rate manuell einstellbar sind. Für die automatische Steuerung und/oder Regelung des Argon-Vorlaufs und/oder der für die APC erforderlichen Argon Flow-Rate ist die Hardware vorzugsweise zusätzlich mit Steuerungs- und/oder Regelungselementen St ausgestattet. In weiterer Ausgestaltung der Erfindung ist die Hardware zusätzlich mit elektronischen Speichern und Prozessoren ausgestattet, welche eine Programmierung und entsprechende automatishe Steuerung und/oder Regelung des Argon-Vorlaufs und/oder des für die APC erforderlichen Argon-Flows ermöglichen.

Auf die beiliegenden Zeichnungen wird hiermit verwiesen.

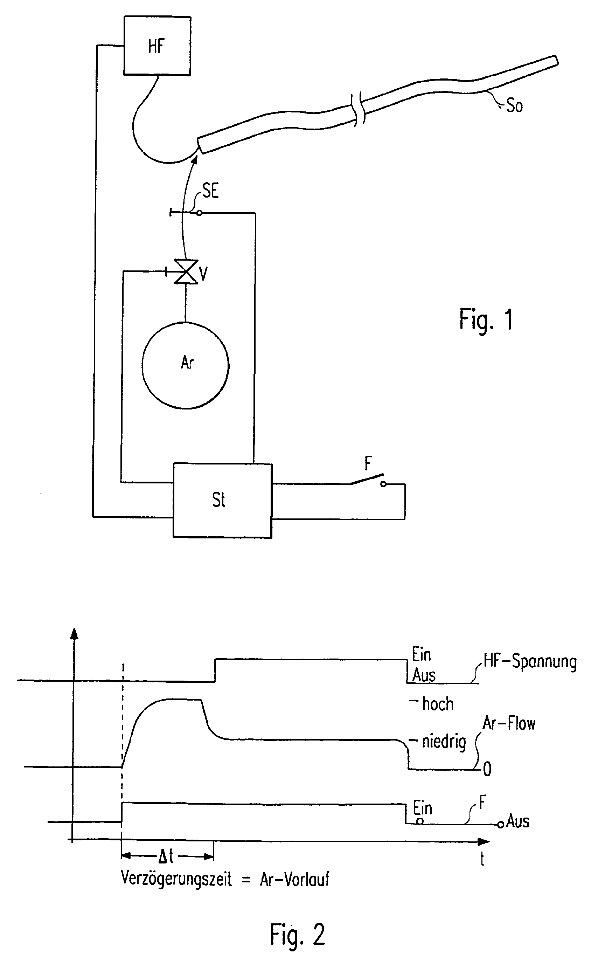

## Patentansprüche

1. Einrichtung für die Argon-Plasma-Koagulation APC, umfassend eine HF-Quelle (HF) und eine Quelle für Argon-Gas (Ar) zum Zuführen eines hochfrequenten elektrischen Wechselstroms und eines Edelgases (Ar) zu einem APC-Instrument, sowie eine Steuerung (St),
**dadurch gekennzeichnet, dass**
die Steuerung (St) derart mit der HF-Quelle (HF) und der Argon-Gasquelle (Ar) verbunden und ausgebildet ist, dass nach Aktivieren der Einrichtung für die APC eine definierte Menge Edelgas aus dem APC-Instrument ausströmt, bevor die Hochfrequenzspannung eingeschaltet wird oder einschaltbar ist.

2. Einrichtung nach Anspruch 1,
**dadurch gekennzeichnet, dass**
die Steuerung (St) derart ausgebildet ist, dass die definierte Menge voreinstellbar ist.

3. Einrichtung nach einem der vorhergehenden Ansprüche,
**dadurch gekennzeichnet, dass**
die Steuerung (St) derart ausgebildet ist, dass die vorbestimmte Menge anhand von variablen Randbedingungen einstellbar ist.

4. Einrichtung nach Anspruch 3,
**dadurch gekennzeichnet, dass**
die Steuerung (St) derart ausgebildet ist, dass die eingestellte Menge umso größer ist, je länger die Pausen zwischen zwei Aktivierungsintervallen und/oder je kürzer vorhergehende Aktivierungsintervalle waren.

5. Einrichtung nach Anspruch 1,
**dadurch gekennzeichnet, dass**
die Steuerung (St) derart ausgebildet ist, dass während eines Zeitintervalls vom Aktivieren der Einrichtung für die APC bis zum Einschalten der Hochfrequenzspannung ein akustisches und/oder visuelles Signal generiert wird, welches einen Anwender darüber informiert, dass die Hochfrequenzspannung noch nicht eingeschaltet ist bzw. noch nicht eingeschaltet werden kann.

6. Einrichtung nach Anspruch 1,
**dadurch gekennzeichnet, dass**
die Steuerung (St) derart ausgebildet ist, dass die Hochfrequenzspannung nicht eingeschaltet wird bzw. nicht einschaltbar ist, wenn die definierte Menge noch nicht aus dem APC-Instrument geströmt ist.

7. Einrichtung nach Anspruch 1,
**dadurch gekennzeichnet, dass**
die Steuerung (St) derart ausgebildet ist, dass das Edelgas nach einem Aktivieren der Einrichtung für die APC und vor einem Einschalten der Hochfrequenzspannung mit einer höheren Strömungsrate ausströmt als nach dem Einschalten der Hochfrequenzspannung während eines APC-Vorganges.

8. Verfahren zum Betreiben einer Einrichtung für die Argon-Plasma-Koagulation (APC),
**dadurch gekennzeichnet, dass**
nach Aktivieren der Einrichtung eine definierte Menge Edelgas aus einem zur Einrichtung gehörigen APC-Instrument ausströmt, bevor eine Hochfrequenzspannung zum Ionisieren des Edelgases eingeschaltet wird oder einschaltbar ist.

## Claims

1. Apparatus for argon-plasma coagulation APC, comprising an HF source (HF) and a source of argon gas (Ar), to supply a high-frequency electrical alternating current and a noble gas (Ar) to an APC instrument, as well as a control means (Co),
**characterized in that** the control means (Co) is connected to the HF source (HF) and the argon-gas source (Ar) and designed in such a way that after activation of the apparatus for APC a specified amount of noble gas flows out of the APC instrument before the high-frequency voltage is switched on, or can be switched on.

2. Apparatus according to Claim 1,
**characterized in that** the control means (Co) is designed such that the specified amount can be adjusted in advance.

3. Apparatus according to one of the preceding claims,
**characterized in that** the control means (Co) is designed such that the prespecified amount can be adjusted with reference to variable boundary conditions.

4. Apparatus according to Claim 3,
**characterized in that** the control means (Co) is designed such that the adjusted amount is greater, the longer the pauses between activation intervals and/or the shorter the preceding activation intervals have been.

5. Apparatus according to Claim 1,
**characterized in that** the control means (Co) is designed such that during the time interval from activation of the apparatus for argon plasma coagulation until the high-frequency voltage is turned on, an acoustic and/or visual signal is generated, which informs the user that the high-frequency voltage has not yet been turned on, or that it cannot yet be turned on.

6. Apparatus according to Claim 1,
**characterized in that** the the control means (Co) is designed such that the high-frequency voltage is not or cannot be turned on as long as the specified amount has not flowed out of the APC instrument.

7. Apparatus according to Claim 1,
**characterized in that** the the control means (Co) is designed such that the rate at which the noble gas flows out is higher after activation of the apparatus for APC but before the high-frequency voltage is turned on than it is after the high-frequency voltage has been turned on during an APC procedure.

8. Method of operating an apparatus for argon plasma coagulation (APC),
**characterized in that** after activation of the apparatus a specified amount of noble gas flows out of an APC instrument belonging to the apparatus before a high-frequency voltage to ionize the noble gas is switched on, or can be switched on.

## Revendications

1. Dispositif pour la coagulation par plasma d'argon APC, comportant une source de haute fréquence (HF) et une source de gaz argon (Ar) pour acheminer un courant alternatif électrique à haute fréquence et un gaz rare (Ar) vers un instrument d'APC, ainsi qu'une commande (St),
**caractérisé en ce que**
la commande (St) est reliée à la source de haute fréquence (HF) et à la source de gaz argon (Ar) et est réalisée de telle sorte que, après l'activation du dispositif pour l'APC, une quantité définie de gaz rare afflue hors de l'instrument d'APC, avant que la tension à haute fréquence soit connectée ou puisse être connectée.

2. Dispositif selon la revendication 1, **caractérisé en ce que** la commande (St) est réalisée de telle sorte que la quantité définie peut être prédéterminée.

3. Dispositif selon l'une des revendications précédentes, **caractérisé en ce que** la commande (St) est réalisée de telle sorte que la quantité prédéterminée peut être réglée en fonction de conditions limites variables.

4. Dispositif selon la revendication 3, **caractérisé en ce que** la commande (St) est réalisée de telle sorte que la quantité réglée est d'autant plus grande que les pauses entre deux intervalles d'activation sont longues et/ou que les intervalles d'activation précédents étaient courts.

5. Dispositif selon la revendication 1, **caractérisé en ce que** la commande (St) est réalisée de telle sorte que pendant un intervalle de temps depuis l'activation du dispositif pour l'APC jusqu'à la connexion de la tension de haute fréquence est généré un signal acoustique et/ou visuel, qui signale à un utilisateur que la tension de haute fréquence n'est pas encore connectée ou ne peut pas encore être connectée.

6. Dispositif selon la revendication 1, **caractérisé en ce que** la commande (St) est réalisée de telle sorte que la tension de haute fréquence n'est pas connectée ou ne peut pas être connectée lorsque la quantité définie n'a pas encore afflué hors de l'instrument d'APC.

7. Dispositif selon la revendication 1, **caractérisé en ce que** la commande (St) est réalisée de telle sorte que, après une activation du dispositif pour l'APC et avant la connexion de la tension de haute fréquence, le gaz rare afflue avec un taux d'écoulement plus élevé qu'après la connexion de la tension de haute fréquence pendant un processus d'APC.

8. Procédé d'utilisation d'un dispositif pour la coagulation par plasma d'argon (APC), **caractérisé en ce que**, après l'activation du dispositif, une quantité définie de gaz rare afflue hors de l'instrument d'APC associé au dispositif, avant qu'une tension de haute fréquence pour l'ionisation du gaz rare soit connectée ou puisse être connectée.
